# EUROPEAN PATENT APPLICATION

(11) **EP 3 466 923 A1**
(43) Date of publication of application: **10.04.2019**
(21) Application number: 17195494.4
(22) Date of filing: 09.10.2017
(51) Int. Cl.: C07C 231/12, C07C 231/24, C07C 233/18

(54) **PROCESS FOR THE PREPARATION OF AGOMELATINE IN CRYSTALLINE FORM**

(71) Applicant: KRKA, d.d., Novo mesto, 8501 Novo mesto (SI)
(72) Inventor: Kidemet, Davor, 42000 Varazdin (HR); Gosak, Kaja, 3000 Celje (SI); Zupancic, Blaz, 1000 Lubljana (SI); Benkic, Primoz, 1000 Lubljana (SI); Pavlin, Darja, 8351 Straza pri Novem mestu (SI); Kolenc, Ivanka, 8000 Novo mesto (SI)
(74) Representative: Hoefer & Partner Patentanwälte mbB

(57) **Abstract**

The present invention pertains to a process for the preparation of polymorph form X of agomelatine, which comprises providing agomelatine, and crystallizing agomelatine in the presence of at least one of an acid and a salt thereof, and to a polymorph form of agomelatine.

## Description

### TECHNICAL FIELD OF THE INVENTION

The present invention relates to a process for the preparation of agomelatine in polymorphic Form X, which comprises crystallizing agomelatine in the presence of acid, as well as to crystalline agomelatine obtainable by this process.

### BACKGROUND OF THE INVENTION

Agomelatine provides an advantageous concept for antidepressant treatment. The agomelatine molecule shows a pharmacological mechanism of action, which provides melatonin MT1 and MT2 agonist properties in combination with a serotonin 5-HT_{2C} antagonist effect.

Agomelatine can be used in particular for the treatment of major depressive disorder. The chemical name of agomelatine is *N*[2-(7-methoxy-1-naphthyl)ethyl] acetamide. The chemical structure of agomelatine is presented by the following Formula (A):

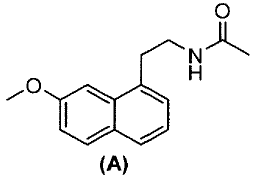

Agomelatine is a highly permeable drug, but is only very slightly soluble in aqueous solution over the physiological pH range.

Furthermore, Agomelatine exhibits polymorphism. EP-B-0 447 285 discloses a process for the preparation of agomelatine, which is in crystalline form I. Crystalline Form I may convert into more stable crystalline Form II during formulation processes. Furthermore, several polymorphs of agomelatine are known from literature such as crystalline Form IV disclosed in EP-A-1 752 444, Crystalline Form V disclosed in EP-B-1 752 443, crystalline Form VI disclosed in EP-A-2 058 296, crystalline Form VII disclosed in EP-A- 2690087, and crystalline form X disclosed in EP- B- 2 474 522 and WO 2011/154140 A2. Furthermore, Asian Journal of Pharmaceutical Sciences 8(2013) 181-190 describes crystalline Form I, II and III of agomelatine.

Metastable polymorphic forms can transform into thermodynamically more stable forms. It is therefore necessary to monitor polymorphic transformation during all stages of preparation, dosage form production, and storage for ensuring a reproducible bioavailability. In addition, a change of the polymorphic form may cause clinical failures. It is therefore crucial that transformation of the agomelatine polymorphic form during preparation, as well as during dosage form production and shelf life is prevented or minimized.

Although several approaches for preparing agomelatine have been described in the art, there still exists a need for further processes for preparing agomelatine in a crystalline form which provides in combination an advantageous solubility, a good bioavaialbility, and an advantageous stability. Especially, the process should provide agomelatine having a reduced tendency for undesired polymorphic transformation. Furthermore, the process should allow obtaining a crystalline form having a good filterability. Moreover, this process for preparing agomelatine should be cost-effective and time-effective. Furthermore, the process should not require complicated devices, should not be energy-intensive, and should be essentially environmentally friendly, and appropriate for the production of agomelatine on industrial scale.

### SUMMARY OF THE INVENTION

According to a first aspect, there is provided a process for the preparation of polymorph form X of agomelatine of formula (A) 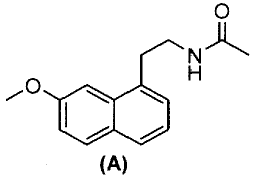 said process comprising:
providing agomelatine of formula (A),
crystallizing agomelatine of formula (A) in the presence of at least one of an acid and a salt thereof,
said crystallizing of agomelatine of formula (A) in the presence of at least one of an acid and a salt thereof comprising:
   a) dissolving agomelatine in a first solvent to obtain a solution, said first solvent being or comprising an organic solvent,
   b) preparing a mixture comprising
      (i) at least one of an acid and a salt thereof, and
      (ii) a second solvent,
         the at least one of an acid and a salt thereof being dissolved in the second solvent, and
         the second solvent being water or a solvent mixture comprising water,
   c) adjusting the temperature of the mixture comprising (i) at least one of an acid and a salt thereof and (ii) a second solvent to a temperature in the range from 15°C to 40°C,
   d) combining the solution comprising agomelatine with the mixture having a temperature in the range from 15°C to 40°C and comprising i) at least one of an acid and a salt thereof, and ii) a second solvent,
   e) isolating crystals of polymorph form X of agomelatine, and
   f) optionally drying the isolated crystals of polymorph form X of agomelatine.

According to a further aspect, there is provided a polymorph form X of agomelatine of formula (A) characterised by Δ H (J/g) value for form II of below 60, preferably below 30, more preferably below 20, even more preferably below 10, as determined by DSC method at heating rate 1°C/min. Optionally, this polymorph form X of agomelatine is obtainable according to the process for the preparation of polymorph form X of agomelatine of the present invention (especially according to the process e.g. defined herein above in the first aspect of the present invention).

According to a further aspect, there is provided a polymorph form X of agomelatine of formula (A) obtainable according to the process for the preparation of polymorph form X of agomelatine of the present invention (especially according to the process e.g. defined herein above in the first aspect of the present invention).

### BRIEF DESCRIPTION OF THE FIGURES

Fig. 1 shows a Differential Scanning Calorimeter thermogram of polymorph form X of agomelatine as prepared in Example 9.
Fig. 2 shows a Differential Scanning Calorimeter thermogram of polymorph form X of agomelatine as prepared in Example 10.
Fig. 3 shows a Differential Scanning Calorimeter thermogram of polymorph form X of agomelatine as prepared in Example 11.
Fig. 4 shows a Differential Scanning Calorimeter thermogram of polymorph form X of agomelatine as prepared in Reference Example 2. In Figures 1 to 4, the horizontal axis represents the temperature in °C, the vertical axis having mW as unit.

### DETAILED DESCRIPTION OF THE PRESENT INVENTION

The present inventors provide a process for preparation of crystalline agomelatine which solves the before-mentioned problems. Surprisingly, this process allows the preparation of crystalline agomelatine in high purity, good yields and having an advantageous stability, as well as an advantageous filterability. The process of the present invention may be carried out on industrial scale. Furthermore, the process of the present invention does not require complicated devices.

The present inventors carried out numerous experiments repeating prior art processes for preparing agomelatine in crystalline form. Repeating prior art processes for preparing agomelatine failed, however, to provide an agomelatine form X in stable polymorphic form. The polymorphs obtainable by repeating prior art processes partially or completely convert into a thermodynamically more stable form, especially into Form II, immediately after production or under storage conditions, as can be simulated by stability testing experiments. The prior art processes turned out to be not reproducible and robust, and provided agomelatine with variable stability, and thus provided agomelatine with variable physical and chemical properties.

Surprisingly, the present inventors now developed a process for the preparation of polymorph form X of agomelatine, which comprises crystallizing agomelatine of formula (A) in the presence of at least one of an acid and a salt thereof. The process of the present invention is a robust and reliable process with high reproducibility, especially in terms of obtaining stable polymorphic form X as determined by Δ H (J/g) value.

The process for the preparation of polymorph form X of agomelatine of formula (A) 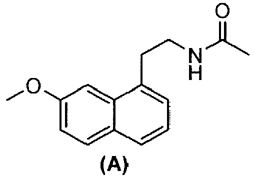 comprises:
providing agomelatine of formula (A), and
crystallizing agomelatine of formula (A) in the presence of at least one of an acid and a salt thereof. Said crystallizing of agomelatine of formula (A) in the presence of at least one of an acid and a salt thereof comprises:
   a) dissolving agomelatine in a first solvent to obtain a solution, said first solvent being or comprising an organic solvent,
   b) preparing a mixture comprising
      (i) at least one of an acid and a salt thereof, and
      (ii) a second solvent, the at least one of an acid and a salt thereof being dissolved in the second solvent, and the second solvent being water or a solvent mixture comprising water,
   c) adjusting the temperature of the mixture comprising (i) at least one of an acid and a salt thereof and (ii) a second solvent to a temperature in the range from 15°C to 40°C,
   d) combining the solution comprising agomelatine with the mixture having a temperature in the range from 15°C to 40°C and comprising i) at least one of an acid and a salt thereof, and ii) a second solvent,
   e) isolating crystals of polymorph form X of agomelatine, and
   f) optionally drying the isolated crystals of polymorph form X of agomelatine.

Step a) of the process of the present invention can be or can comprise dissolving agomelatine in a first solvent to obtain a solution, said first solvent being or comprising an organic solvent. Agomelatine may be prepared according to any one of various processes known in the art. For example, the skilled person may prepare agomelatine according to the process described herein or may prepare agomelatine according to processes described in one of the documents mentioned herein, such as e.g. according to EP-B-0 447 285, EP-A-1 752 444, EP-B-1 752 443, EP-A-2 058 296, EP-A-2 690 087, EP-B-2 474 522.

The first solvent can be selected from the group consisting of hydrocarbons, in particular benzene, optionally substituted with one or two or three substituents which each can be independently selected from C₁-C₂-alkyl, such as toluene, xylenes (in particular ortho- or meta- or para xylene and mixtures thereof),
ketones, such as acetone, methyl ethyl ketone, methyl isobutyl ketone, 2-butanone, alcohols, preferably alcohols comprising one to five carbon atoms, such as methanol, ethanol, propanols, e.g. isopropyl alcohol, butanols, e.g. n-butanol, isobutyl alcohol, tertiary butyl alcohol,
sulfoxides, such as dimethyl sulfoxide (DMSO),
ethers, such as 1,4-dioxane, tetrahydrofuran, diisopropylether; carbon acid amides, such as N,N-dimethyl formamide (DMF), dimethyl acetamide, N-methyl pyrrolidone (MP), and mixtures thereof.

In particular, the first solvent can be a solvent selected from the group consisting of dimethylformamide (DMF), toluene, xylene, and mixtures thereof.

In particular, the first solvent can be not an organic acid, especially the first solvent can be not an aliphatic acid; further especially can be not a compound selected from compounds of formula R^{al}-COOH, R^{al} being e.g. H or alkyl, preferably R^{al} being selected from H, methyl, ethyl and propyl.

Dissolving agomelatine to obtain a solution can comprise adding agomelatine to the first solvent, and removing undissolved agomelatine, e.g. by filtration.

An organic solvent can be in particular an organic compound or a mixture of organic compounds. An organic compound can be in particular a compound comprising at least one carbon atom. In a preferred embodiment, a solvent can be a solvent which is liquid at a temperature of 25°C (especially 25°C, at a pressure of 101 325 Pa) or higher.

The process of the present invention further comprises b) preparing a mixture comprising (i) at least one of an acid and a salt thereof, and (ii) a second solvent, the at least one of an acid and a salt thereof being dissolved in the second solvent, and the second solvent being water or a solvent mixture comprising water.

In particular, the second solvent can be a solvent selected from the group consisting of water and solvent mixtures comprising water. The solvent mixture comprising water can further comprise organic solvent. In the solvent mixture comprising water, the weight ratio (organic solvent: water) can be less than 1:2, preferably less than 1:5, more preferably less than 1:10.

Said organic solvent present in the solvent mixture comprising water will be referred to hereinafter also as "organic third solvent". The organic third solvent can be selected from the group consisting of hydrocarbons, in particular benzene, optionally substituted with one or two or three substituents which each can be independently selected from C₁-C₂-alkyl, such as toluene, xylene (in particular can be selected from ortho-xylene, meta- xylene, para xylene and mixtures thereof), C₅-C₈-cycloalkanes, optionally substituted with one or two or three substituents which each can be independently selected from C₁-C₂-alkyl, such as cyclohexane, C₅-C₉-alkanes, such as n-hexane,
ketones, such as acetone, methyl ethyl ketone, methyl isobutyl ketone, 2-butanone, alcohols, preferably alcohols comprising one to five carbon atoms, such as methanol, ethanol, propanols, e.g. isopropyl alcohol, butanols, e.g. n-butanol, isobutyl alcohol, tertiary butyl alcohol,
sulfoxides, such as dimethyl sulfoxide (DMSO),
ethers, such as 1,4-dioxane, tetrahydrofuran, diisopropylether; carbon acid amides, such as N,N-dimethyl formamide (DMF), dimethyl acetamide, N-methyl pyrrolidone (MP), and mixtures thereof.

Especially, in the process of the present invention, especially in step c) thereof, the organic solvent present in the solvent mixture comprising water (which is also referred to herein as "organic third solvent") can be the same solvent as the second solvent.

In the context of the present invention, the term "solvent" can have the meaning of a single solvent or a mixture of solvents. Steps a) to e) of the process of the present invention can be carried out with or without intermediate steps. In one embodiment, steps a) to e) of the process of the present invention can be carried out directly one after another.

Step c) can be or can comprise adjusting the temperature of the mixture comprising (i) at least one of an acid and a salt thereof and (ii) a second solvent to a temperature in the range from 15°C to 40°C, optionally in the range from 17°C to 35°C, further optionally in the range from 20°C to 30°C, still further optionally in the range from 23°C to 27°C, still further optionally in the range from 24-26°C, especially about 25°C. Optionally, step c) can further comprise adjusting the temperature of the solution comprising agomelatine to a temperature in the range from 15°C to 40°C, optionally in the range from 17°C to 35°C, further optionally in the range from 20°C to 30°C, still further optionally in the range from 23°C to 27°C, still further optionally in the range from 24-26°C, especially about 25°C.

In an embodiment, step c) can be or can comprise adjusting both the temperature of the solution comprising agomelatine and the temperature of the mixture comprising (i) at least one of an acid and a salt thereof and (ii) a second solvent to a temperature in the range from 15°C to 40°C, optionally in the range from 17°C to 35°C, further optionally in the range from 20°C to 30°C, still further optionally in the range from 23°C to 27°C, still further optionally in the range from 24-26°C, especially about 25°C.

In a subsequent step d), the solution comprising agomelatine can be combined with the mixture comprising i) at least one of an acid and a salt thereof, and ii) a second solvent, said mixture having a temperature in the range from 15°C to 40°C, optionally in the range from 17°C to 35°C, further optionally in the range from 20°C to 30°C, still further optionally in the range from 23°C to 27°C, still further optionally in the range from 24-26°C, especially about 25°C; optionally, the solution comprising agomelatine can have a temperature in the range from 15°C to 40°C, optionally in the range from 17°C to 35°C, further optionally in the range from 20°C to 30°C, still further optionally in the range from 23°C to 27°C, still further optionally in the range from 24-26°C, especially about 25°C.

In an embodiment, step d) can be combining the solution comprising agomelatine with the mixture comprising i) at least one of an acid and a salt thereof, and ii) a second solvent, wherein both the solution comprising agomelatine and the mixture comprising i) at least one of an acid and a salt thereof, and ii) a second solvent can have a temperature in the range from 15°C to 40°C, optionally in the range from 17°C to 35°C, further optionally in the range from 20°C to 30°C, still further optionally in the range from 23°C to 27°C, still further optionally in the range from 24-26°C, especially about 25°C.

During combining step d), the temperature of the mixture resulting from combining the solution comprising agomelatine with the mixture comprising i) at least one of an acid and a salt thereof, and ii) a second solvent can have a temperature in the range from 15°C to 40°C, optionally in the range from 17°C to 35°C, further optionally in the range from 20°C to 30°C, still further optionally in the range from 23°C to 27°C, still further optionally in the range from 24-26°C, especially about 25°C.

Optionally, during the step of combining the mixture resulting from combining the solution comprising agomelatine with the mixture comprising i) at least one of an acid and a salt thereof, and ii) a second solvent can be subjected to agitation, e.g. stirring.

Combining step d) can be adding the solution comprising agomelatine to the mixture comprising i) at least one of an acid and a salt thereof, and ii) a second solvent and having a temperature preferably in the range from 15°C to 40°C, optionally in the range from 17°C to 35°C, further optionally in the range from 20°C to 30°C, still further optionally in the range from 23°C to 27°C, still further optionally in the range from 24-26°C, especially about 25°C. The adding of the solution comprising agomelatine can be adding the solution comprising agomelatine in two or more portions, especially dropwise, to the mixture comprising i) at least one of an acid and a salt thereof, and ii) a second solvent. During the adding of the solution comprising agomelatine, the mixture comprising i) at least one of an acid and a salt thereof, and ii) a second solvent, can be preferably subjected to agitation, especially subjected to stirring.

In particular, the mixture comprising i) at least one of an acid and a salt thereof, and ii) a second solvent can comprise said at least one of an acid and a salt thereof and said second solvent in a weight ratio (weight amount of said at least one of an acid and a salt thereof) : (weight amount of said second solvent) of less than 1 : 6, optionally of less than 1 : 8, further optionally of less than 1:12.

Furthermore, the solution comprising agomelatine (which is obtained in step a) by dissolving agomelatine in the first solvent and which can be combined in step d) with the mixture comprising i) at least one of an acid and a salt thereof and ii) a second solvent) can comprise agomelatine and first solvent in a weight ratio (agomelatine : first solvent) in the range from 1:2.5 to 1:20, preferably from 1:3 to 1:10, most preferably 1:5.

Furthermore, the solution comprising agomelatine (which is obtained in step a) by dissolving agomelatine in DMF (as first solvent) and which can be combined in step d) with the mixture comprising i) at least one of an acid and a salt thereof and ii) a second solvent) can comprise agomelatine and DMF in a weight ratio (agomelatine : DMF) which can be in the range from 1:2.5 to 1:20, preferably 1:3 to 1:10, most preferably 1:5.

In the step of combining the solution comprising agomelatine with the mixture comprising i) at least one of an acid and a salt thereof, and ii) a second solvent, said solution comprises agomelatine in such an amount and said mixture comprises said second solvent in such an amount that the weight ratio (agomelatine : second solvent), especially the weight ratio (agomelatine : water), can be in the range from 1:50 to 1:1000, preferably from 1:60 to1:300, more preferably from 1:75 to 1:150, most preferably from 1:100; preferably said second solvent can be water. The mixture obtained after having completed combining step d) comprises agomelatine and said second solvent in such amounts that the weight ratio (agomelatine : water), especially the weight ratio (agomelatine : water), can be in the range from 1:50 to 1:1000, preferably from 1:60 to1:300, more preferably from 1:75 to 1:150, most preferably 1:100; preferably said second solvent can be water.

In the step of combining the solution comprising agomelatine with the mixture comprising i) at least one of an acid and a salt thereof, and ii) a second solvent, said solution comprises agomelatine in such an amount and said mixture comprises said at least one of an acid and a salt thereof in such an amount that the molar ratio (agomelatine : said at least one of an acid and a salt thereof) can be in the range from 1: 0.05 to 1:1.

In one embodiment, the term "at least one of an acid and a salt thereof" can have the meaning of an acid. The term "acid" can encompass one acid or a mixture of acids. In another embodiment, the term "at least one of an acid and a salt thereof" has the meaning of salt of an acid. The term "salt of an acid" can encompass one salt or a mixture of salts.

When the solution containing agomelatine is contacted with the mixture comprising i) at least one of an acid and a salt thereof, and ii) a second solvent, agomelatine precipitates. Optionally, it is possible to allow completion of the crystallization after having combined the solution containing agomelatine with the mixture comprising i) at least one of an acid and a salt thereof, and ii) a second solvent. For example, it is possible to allow completion of the crystallization for a time span of e.g. up to 24 h, preferably up to 30 minutes.

In particular, after having combined the solution comprising agomelatine with the mixture comprising i) at least one of an acid and a salt thereof, and ii) a second solvent, the mixture obtained by combining the solution comprising agomelatine with the mixture comprising i) at least one of an acid and a salt thereof and ii) a second solvent, can be subjected to a temperature in the range from 15°C to 40°C, optionally in the range from 17°C to 35°C, further optionally in the range from 20°C to 30°C, still further optionally in the range from 23°C to 27°C, still further optionally in the range from 24-26°C, especially about 25°C, optionally under agitation (e.g. stirring), for example for a time span of e.g. up to 24 h, preferably up to 30 minutes.

Especially, the mixture obtained after combining step d) can be subjected to a temperature in the range from 15°C to 40°C, optionally in the range from 17°C to 35°C, further optionally in the range from 20°C to 30°C, still further optionally in the range from 23°C to 27°C, still further optionally in the range from 24-26°C, especially about 25°C, until starting the step of isolating crystals of polymorph form X of agomelatine.

In an embodiment of the process of the present invention, during or after step d) of combining the solution comprising agomelatine with the mixture comprising i) at least one of an acid and a salt thereof, and ii) a second solvent agomelatine seed crystals (especially seed crystals having polymorph form X of agomelatine) can be added; preferably agomelatine seed crystals can be added in a weight amount which is between less than 3% by weight, e.g. between 0.005% by weight and less than 3% by weight, preferably less than 2% by weight, of the total weight amount of agomelatine dissolved in the solution comprising agomelatine (especially the solution which is combined with the mixture in step d)).

In particular, the combining step can be combining the solution comprising agomelatine (optionally, said solution comprising agomelatine may be enriched with (undissolved) agomelatine seed crystals before combining step d)) with the mixture comprising i) at least one of an acid and a salt thereof, ii) a second solvent, and further comprising iii) agomelatine seed crystals (especially seed crystals having polymorph form X of agomelatine); preferably, the total weight amount of seed crystals being less than 3% by weight, e.g. between 0.005% by weight and less than 3% by weight, preferably less than 2% by weight, of the total weight amount of agomelatine dissolved in the solution comprising agomelatine (especially the solution which is combined with the mixture in step d)).

Seed crystals can be in particular seed crystals having polymorph form X of agomelatine. These seed crystals may be obtained according to crystallization procedures described herein or according to procedures known in the art, e.g. described in EP-B-2 474 522 A1 or WO 2011/154140 A2.

The term "seed crystals" as used herein, encompasses two or more seed crystals, as well as one single seed crystal.

The present inventors found that seeding the mixture comprising an acid and a second solvent can facilitate the crystallization of the desired polymorph Form X in high purity and can enhance physical stability of polymorph Form X. Preferably, seeding is performed with less than 3% by weight of agomelatine seeds, preferably less than 2% by weight, based on the weight amount of agomelatine dissolved (especially based on the weight amount of agomelatine dissolved in the solution comprising agomelatine (for use in step d)). Seeds of agomelatine used can be polymorph form X of agomelatine having Δ H (J/g) value of less than 60 (J/g), more preferred are seeds with Δ H (J/g) value of less than 30 (J/g), even more preferred less than 10 (J/g) (Δ H (J/g) value can be determined by DSC method, especially at a heating rate of 1°C /minute).

In particular, steps c) and d) can be:
c) adjusting the temperature of the mixture comprising (i) at least one of an acid and a salt thereof and (ii) a second solvent to a temperature in the range from 15°C to 40°C (optionally in the range from 17°C to 35°C, further optionally in the range from 20°C to 30°C, still further optionally in the range from 23°C to 27°C, still further optionally in the range from 24-26°C, especially about 25°C), and adding seed crystals of polymorph form X of agomelatine to the mixture, and
d) combining the solution comprising agomelatine with the mixture having a temperature in the range from 15°C to 40°C (optionally in the range from 17°C to 35°C, further optionally in the range from 20°C to 30°C, still further optionally in the range from 23°C to 27°C, still further optionally in the range from 24-26°C, especially about 25°C), and comprising i) at least one of an acid and a salt thereof, ii) a second solvent, and (iii) seed crystals of polymorph form X of agomelatine (preferably the total weight amount of seed crystals being less than 3% by weight, e.g. between 0.005% by weight and less than 3% by weight, preferably less than 2% by weight, of the total weight amount of agomelatine dissolved in the solution comprising agomelatine).

In the process of the present invention the at least one of an acid and a salt thereof can be selected from the group consisting of organic acids, inorganic acids, salts of organic acids, salts of inorganic acids, and mixtures thereof. Salt can be in particular salt selected from the group of alkali salts (e.g. sodium salts, potassium salts) of (organic or inorganic) acids, and earth alkali salts of (organic or inorganic) acids, and mixtures thereof. In an embodiment, salt can be selected from alkali salts of organic acids, especially from sodium salts of organic acids.

Preferably, the at least one of an acid and a salt thereof can be selected from compounds comprising one or two or more carboxylic acid moieties, salts (in particular alkali (e.g. sodium salts, potassium salts) or earth alkali salts) of compounds comprising one or two or more carboxylic acid moieties, compounds comprising one or two or more sulfonic acid moieties, salts (in particular alkali (e.g. sodium salts, potassium salts) or earth alkali salts) of compounds comprising one or two or more sulfonic acid moieties, and mixtures thereof.

In particular, the at least one of an acid and a salt thereof can be or comprise one or more selected from the group consisting of 1-hydroxy-2-naphthoic acid and salts thereof, 2,2-dichloroacetic acid and salts thereof, 2-hydroxyethanesulfonic acid and salts thereof, 2-oxoglutaric acid and salts thereof, 4-acetamidobenzoic acid and salts thereof, 4-amino-salicylic acid and salts thereof, acetic acid and salts thereof, adipic acid and salts thereof, ascorbic acid and salts thereof, aspartic acid and salts thereof, benzenesulfonic acid and salts thereof, benzoic acid and salts thereof, camphoric acid and salts thereof, camphor-10-sulfonic acid and salts thereof, capric acid (decanoic acid) and salts thereof, caproic acid (hexanoic acid) and salts thereof, caprylic acid (octanoic acid) and salts thereof, carbonic acid and salts thereof, cinnamic acid and salts thereof, citric acid and salts thereof, cyclamic acid and salts thereof, dodecylsulfuric acid and salts thereof, ethane-1,2-disulfonic acid and salts thereof, ethanesulfonic acid and salts thereof, formic acid and salts thereof, fumaric acid and salts thereof, galactaric acid and salts thereof, gentisic acid and salts thereof, glucoheptonic acid and salts thereof, gluconic acid and salts thereof, glucuronic acid and salts thereof, glutamic acid and salts thereof, glutaric acid and salts thereof, glycerophosphoric acid and salts thereof, glycolic acid and salts thereof, hippuric acid and salts thereof, hydrobromic acid and salts thereof, hydrochloric acid and salts thereof, isobutyric acid and salts thereof, lactic acid and salts thereof, lactobionic acid and salts thereof, lauric acid and salts thereof, maleic acid and salts thereof, malic acid and salts thereof, malonic acid and salts thereof, mandelic acid and salts thereof, methanesulfonic acid and salts thereof, naphthalene-1,5-disulfonic acid and salts thereof, naphthalene-2-sulfonic acid and salts thereof, nicotinic acid and salts thereof, nitric acid and salts thereof, oleic acid and salts thereof, oxalic acid and salts thereof, palmitic acid and salts thereof, pamoic acid and salts thereof, phosphoric acid and salts thereof, proprionic acid and salts thereof, pyroglutamic acid and salts thereof, salicylic acid and salts thereof, sebacic acid and salts thereof, stearic acid and salts thereof, succinic acid and salts thereof, sulfuric acid and salts thereof, tartaric acid and salts thereof, thiocyanic acid and salts thereof, toluenesulfonic acid and salts thereof, undecylenic acid and salts thereof, and mixtures thereof; wherein in particular the salts can be alkali salts (e.g. sodium salts, potassium salts) or earth alkali salts of the before-mentioned acids.

Furthermore, the at least one of an acid and a salt thereof can be selected from the group consisting of compounds of formula (Ac1), compounds of formula (Ac2), compounds of formula (Ac3), compounds of formula (Ac4), salts of compounds of formula (Ac1), salts of compounds of formula (Ac2), salts of compounds of formula (Ac3), salts of compounds of formula (Ac4), and mixtures thereof:

R¹-COOH (Ac1)

R²-SO₃H (Ac2)

HOOC-R³-COOH (Ac3)

HO₃S-R⁴-SO₃H (Ac4)

wherein R¹ and R² can be independently selected from linear or branched C₁-C₁₀ alkyl, (especially linear or branched C₂-C₉ alkyl), optionally substituted with one or more substituents selected from -OH, -COOH, -SO₃H, and C₁-C₄-alkyl; C₆-C₁₂ cycloalkyl, optionally substituted with one or more substituents selected from -OH, -COOH, -SO₃H, and C₁-C₄-alkyl; linear or branched C₁-C₁₀ alkenyl, optionally substituted with one or more substituents selected from -OH, -COOH, -SO₃H, and C₁-C₄-alkyl; C₆-C₁₂ aryl, optionally substituted with one or more substituents selected from -OH, -COOH, -SO₃H, and C₁-C₄-alkyl;
wherein R³ and R⁴ can be independently selected from linear or branched C₁-C₁₀ alkylene (preferably linear or branched C₁-C₆ alkylene), optionally substituted with one or more substituents selected from -OH, -COOH, -SO₃H, and C₁-C₄-alkyl; C₆-C₁₂ cycloalkylene, optionally substituted with one or more substituents selected from -OH, -COOH, -SO₃H, and C₁-C₄-alkyl; and C₆-C₁₂ arylene, optionally substituted with one or more substituents selected from -OH, -COOH, -SO₃H, and C₁-C₄-alkyl; wherein in particular the salts can be alkali salts (e.g. sodium salts, potassium salts) or earth alkali salts of the before-mentioned acids. Preferably, R³ and R⁴ can be independently selected from linear or branched C₁-C₆ alkylene (especially a C₁ or C₂ or C₃ or C₄ or C₅ alkylene), which is unsubstituted or which is substituted with at least one (e.g. 1, 2, 3, 4) -OH substituent, and is optionally further substituted with at least one -COOH substituent; wherein in particular the salts can be alkali salts (e.g. sodium salts, potassium salts) or earth alkali salts of the before-mentioned acids.

Particularly advantageous results were observed when the at least one of an acid and a salt thereof is selected from the group consisting of acetic acid, tartaric acid, salts (in particular alkali (e.g. sodium, potassium) or earth alkali salts) of acetic acid, such as sodium acetate, salts (in particular alkali (e.g. sodium, potassium) or earth alkali salts) of tartaric acid, hydrochloric acid and mixtures thereof.

Step e) of the process of the present invention is or comprises isolating crystals of polymorph form X of agomelatine. Isolating the crystals may be carried out by any method for isolating crystals known in the art. For example, crystals of agomelatine can be isolated by filtration or by centrifugation. Optionally, the isolated crystals can be washed with second solvent, preferably water, after isolation. Washing of the crystals can be carried out by any method for washing crystals known in the art.

The isolated polymorph form X of agomelatine can have a chemical purity of preferably 98% or more, more preferably 99% by weight or more, in particular 99.5% by weight or more, such as 99.7% by weight or more. The obtained polymorph form X of agomelatine can have a polymorph purity of preferably 98% or more, more preferred 99% or more, in particular 99.5% or more, such as 99.7% or more (all %-values in this context are % by weight).

Optional step f) of the process of the present invention is or comprises optionally drying the isolated crystals of polymorph form X of agomelatine. Drying can in particular refer to the removal of solvent residues performed for example by heating to a temperature above room temperature, e.g. above 20°C) (in particular at normal pressure or at a pressure reduced below ambient pressure) or by drying at a pressure reduced below ambient pressure, where the crystals are kept in stationary phase or are continuously moving, e.g. fluid bed drying. The isolated crystals can be dried by subjecting the crystals to e.g. to a temperature which is not above 40°C, preferably is less than 35°C, more preferably less than 30°C, such as a temperature in the range 15°C to 35°C, even more preferred 23 °C to 28 °C.

In particular, four advantageous reaction steps have been developed by the present inventors for preparing agomelatine.

In combination, these reaction steps provide agomelatine. Table 1 below shows an overview of reaction steps (A.) to (D.):

**Table 1**

| |
|---|
| Horner Wadsworth Emmons reaction |
| 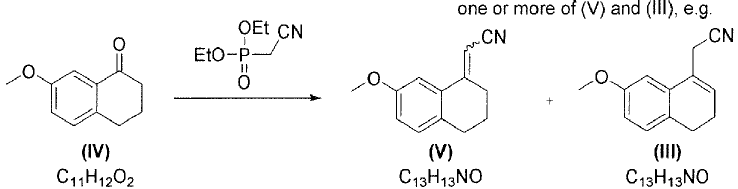 |
| Aromatisation |
| 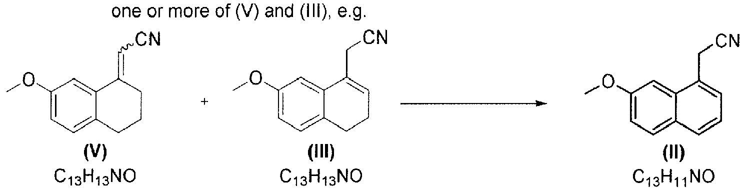 |
| Reduction of nitrile and preparation of HCl salt |
| 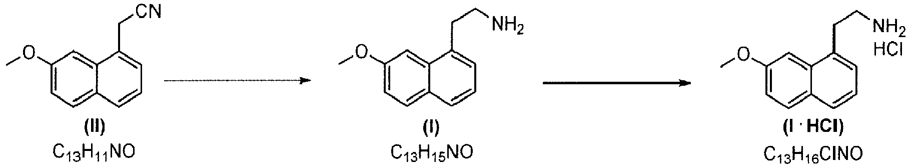 |
| Acetylation |
| 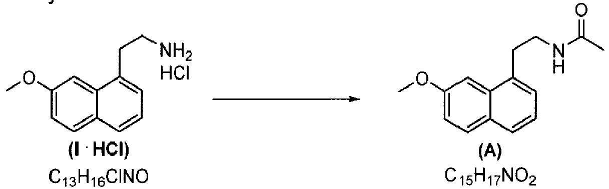 |

A first process step (referred to hereinafter also as step (A.), is or comprises reacting compound of formula (IV) 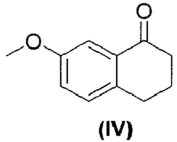 with dialkyl cyanomethylphosphonate, preferably diethyl cyanomethylphosphonate, to obtain one or more of: compound of formula (III) and compound of formula (V)

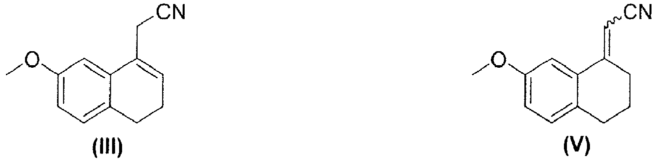

Compound of formula (V) may exist in two forms. In the context of the present application, the term "compound of formula (V)" encompasses the cis-isomer of compound of formula (V), trans-isomer of compound of formula (V), and mixtures consisting of cis-isomer and trans-isomer of compound of formula (V). The term "one or more of: compound of formula (III) and compound of formula (V)" can have the meaning of "compound of formula (III) or compound of formula (V) or mixture of compound of formula (III) and compound of formula (V)".

In particular, a mixture comprising or consisting of compound of formula (III) and compound of formula (V) can be obtained by reacting compound of formula (IV) with diethyl cyanomethylphosphonate.

The reacting of compound of formula (IV) can be carried out in the presence of a base. Reacting compound of formula (IV) with diethyl cyanomethylphosphonate in the presence of a base may advantageously contribute to the reaction of step (A.). The base can be combined with diethyl cyanomethylphosphonate (or a solution or dispersion thereof in solvent) before or during or after (preferably before) the combining of diethyl cyanomethylphosphonate with compound of formula (IV).

The base can be selected from the group of organic and inorganic bases, and mixtures thereof. Preferably, the base can be selected from the group consisting of carbonates, such as alkali carbonates, earth alkali carbonates, especially potassium carbonate (K₂CO₃) or caesium carbonate (CsCO₃), hydrogencarbonates, such as alkali hydrogencarbonates, earth alkali hydrogencarbonates, especially potassium hydrogencarbonate (KHCO₃), hydroxides, such as alkali hydroxides, earth alkali hydroxides, especialy sodium hydroxide, alkali phosphates, such as potassium phosphate, earth alkali phosphates, alkali hydrogenphosphates, such as potassium hydrogenphosphate (K₂HPO₄), earth alkali hydrogenphosphates, sodium hexamethyldisilazane, NaHMDS (sodium hexamethyldisilazide), (in particular mixtures of NaH and NaHDMS (especially NaH in NaHMDS)), NaH (sodium hydride), and mixtures thereof can be used. Especially, potassium carbonate, or caesium carbonate, or potassium phosphate or sodium hexamethyldisilazane can be used as base.

The reacting of compound of formula (IV) can be carried out in a solvent. The solvent can be in particular an organic solvent, especially an inert organic solvent. The solvent (for carrying out the reaction of compound of formula (IV)), can be, but is not limited to, a solvent selected from the group consisting of tetrahydrofuran (THF), 2-methyl-tetrahydrofuran (2-MeTHF), propanols, such as isopropanol, butanols, such as tert-butanol, pentanols, e.g. isoamyl alcohol, sec-butanol, dimethylformamide (DMF), dimethylacetamide (DMAc), bis (2-Methoxyethyl) ether (diglyme), diethylene glycol dibutyl ether (butyl diglyme), cyclopentyl methyl ether, and mixtures thereof. Preferably, the reacting of compound of formula (IV) can be carried out in isopropanol or THF.

In a preferred embodiment, compound of formula (IV) can be reacted with dialkyl cyanomethylphosphonate in isopropanol in the presence of K₂CO₃. Isopropanol and K₂CO₃ are environmentally friendly compounds, as well as are advantageous under toxicological aspects and are compounds which may be advantageously used for the preparation of pharmaceutical active agents, even on industrial scale.

Reacting compound of formula (IV) with diethyl cyanomethylphosphonate in the presence of alkali carbonates, especially potassium carbonate (K₂CO₃) or caesium carbonate (CsCO₃) or of alkali phosphates, such as potassium phosphate (K₃PO₄), can be carried out in a temperature range which is preferably between about 25°C to below the boiling point of solvent used, more preferably between 25°C to 90°C.

In another preferred embodiment, compound of formula (IV) can be reacted with dialkyl cyanomethylphosphonate in THF in the presence of NaHMDS (sodium hexamethyldisilazide). Reacting compound of formula (IV) with diethyl cyanomethylphosphonate in presence of NaHMDS, can be carried out in a temperature range which is preferably between about 0°C to below the boiling point of solvent used, more preferably between about 0°C and 70°C.

The diethyl cyanomethylphosphonate is preferably added in amounts of 1 to 2 (mole) equivalents relative to (one mole equivalent of) the compound of formula (IV), most preferably 1.25 to 1.5 (mole) equivalents. Process step A.), can be or comprise reacting compound of formula (IV) with diethyl cyanomethylphosphonate, wherein diethyl cyanomethylphosphonate and compound of formula (IV) are present in amounts that the ratio (molar amount of diethyl cyanomethylphosphonate) : (molar amount of compound of formula (IV)) is in the range from 1:1 to 2:1, preferably in the range from 1.25:1 to 1.5:1.

The base is preferably added in amounts of 1 to 5 mole equivalents relative to (one mole equivalent of) the compound of formula (IV), most preferably 1.5 to 3 mole equivalents. When the step of reacting compound of formula (IV) is carried out in the presence of a base, the base and the compound of formula (IV) can be preferably present in an amount that the ratio (molar amount of base) : (molar amount of compound of formula (IV)) is in the range from 1:1 to 5:1, preferably in the range from 1.5:1 to 3:1.

Ilustrative of the solvents that can be utilized either singly or in combination are THF, 2-MeTHF, cyclopenthylmethyl ether, isopropanol, tert-butanol, isoamyl alcohol, sec-butanol, DMF, DMAc, diglyme, buthyl diglyme, preferably isopropanol or THF.

In particular, the base can be dissolved or suspended in the solvent to obtain a solution or suspension. Subsequently, in case of alkali carbonates, especially potassium carbonate (K₂CO₃) or caesium carbonate (CsCO₃) or in case of alkali phosphates (K₃PO₄) as base, the obtained solution or suspension can be combined with diethyl cyanomethylphosphonate, for example at a temperature of 0 °C or 30°C, more preferably at a temperature of 20 to 25°C. The so obtained mixture can then be combined with a compound of formula (IV) to obtain a reaction mixture. The obtained reaction mixture can be stirred, for example at a temperature in the range 20°C to 90°C. After completion of the reaction, reaction suspension is filtered and solvent is evaporated. The so obtained mixture can be then extracted with an organic solvent, such as e.g. toluene. After extraction, the aqueous phase and the phase containing the organic phase can be separated, and the organic phase can be washed with water. Finally, the solvent can be removed, e.g. by evaporation. The obtained residue can be used without further purification in the following process step.

Reaction, in the presence of NaHMDS (sodium hexamethyldisilazide) as base: NaHMDS, which can be dissolved or suspended in the solvent to obtain a solution or suspension, can be combined with diethyl cyanomethylphosphonate, for example at a temperature of 8 °C or below, more preferably at a temperature of 5°C or below. The so obtained mixture can then be combined with a compound of formula (IV) (which is preferably dissolved in a solvent) to obtain a reaction mixture. The obtained reaction mixture can be stirred, for example at a temperature in the range 5°C to 30°C. After completion of the reaction, an acidic aqueous solution, such as a solution of acetic acid in water can be added, while preferably keeping the temperature under 35°C. The so obtained mixture can be then extracted with an organic solvent, such as e.g. toluene. After extraction, the aqueous phase and the phase containing the organic phase can be separated, and the organic phase can be washed with water. Finally, the solvent can be removed, e.g. by evaporation. The obtained residue can be used without further purification in the following process step.

In a preferred embodiment of reaction step (A.) is a Horner - Wadsworth - Emmons reaction, e.g. as shown below:

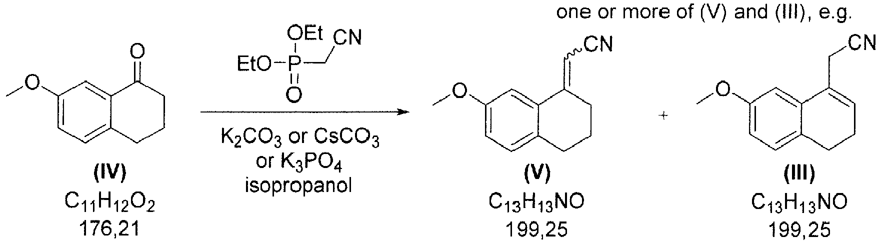

A second process step (referred to hereinafter also as step (B.)), is or comprises subjecting one or more of: compound of formula (III) and compound of formula (V) to an aromatization reaction 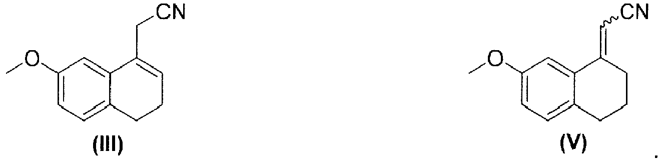 to obtain a compound of formula (II):

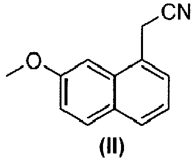

In particular, a mixture comprising or consisting of compound of formula (III) and compound of formula (V) can be subjected to aromatization reaction. Especially, a mixture comprising or consisting of compound of formula (III), cis-isomer of compound of formula (V), and trans-isomer of compound of formula (V) can be subjected to aromatization reaction.

Subjecting one or more of: compound of formula (III) and compound of formula (V) to aromatization reaction can be contacting one or more of: compound of formula (III) and compound of formula (V) with a hydrogenation catalyst, optionally in presence or absence of nitrogen. Preferably, the hydrogenation catalyst can be a hydrogenation catalyst consisting of or comprising palladium, especially palladium on a carrier, e.g. carbon (Pd/C). Palladium on a carrier can contain palladium in an amount of 2 to 12 wt.-%, especially 5 to 10 wt.-%, based on the total weight of palladium and carrier. Palladium on a carrier can be for example palladium on a carrier, wherein the carrier can be selected from the group consisting of carbon, aluminum oxide, barium sulfate, calcium carbonate, strontium carbonate, and mixtures thereof, preferably palladium on a carrier is palladium on carbon containing palladium in an amount of 2 to 12 wt.-%, especially 5 to 10 wt.-%, based on the total weight of palladium and carrier.

In particular, the reaction of one or more of: compound of formula (III) and compound of formula (V), especially the reaction of a mixture of compound of formula (V) (which can be e.g. a mixture of cis- and trans- isomers of compound of formula (V)) and compound of formula (III) (in any ratio) to obtain compound of formula (II) can be carried out in the presence of palladium on a carrier (especially palladium on carbon), optionally in a solvent, and in the presence of or in the absence of hydrogen acceptor. In preferred embodiments the reaction is carried out in the presence of a solvent, or is carried out in the presence of a solvent and a hydrogen acceptor, or is carried out in the presence of a hydrogen acceptor. The hydrogen acceptor may be selected from the group consisting of nitrotoluene (especially o- (ortho-), m- (meta-), p-(para-)nitrotoluene, or mixtures thereof), nitrobenzene, maleic acid, dimethylmaleate, alpha-methylstyrene, tetralin, indene or allyl metacrylate, and mixtures thereof. Preferably, maleic acid or p-nitrotoluene are used. The solvent may be selected from the group consisting of diglyme, butyl diglyme, ethylenglycole, tripropylen glycole, DMF (N,N-dimethyl formamide), dimethylacetamide (DMAC), NMP (*N*-Methyl-2-pyrrolidone), and mixtures thereof, preferably the solvent can be diglyme. In one embodiment, the reaction can be carried out without solvent (especially without organic solvent, further especially without addition of organic solvent). The reaction temperature is below the boiling point of solvent used, preferably between 120 and 150°C. Using diglyme as solvent provides the advantage that diglyme may be separated from the reaction mixture and may be used again (without or with additional purification). The conditions of the aromatization reaction step as described herein offer the advantage of being mild, and avoid drastic aromatisation conditions that are not compatible with the industrial requirements for the purpose of carrying out the synthesis of agomelatine.

Optionally, compound of formula (II) may be crystallized from water, methanol, ethanol, isopropanol and mixtures thereof.

A third process step (referred to hereinafter also as step (C.)), is or comprises reacting compound of formula (II) 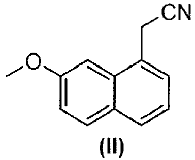 with hydrogen to obtain the compound of formula (I):

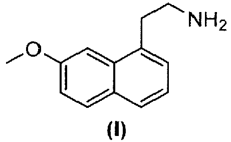

Preferably, a solution or suspension comprising compound of formula (II) and a solvent is reacted with hydrogen; optionally the solution or suspension comprises a base. The solvent, preferably an inert solvent, may be selected from the group consisting of tetrahydrofurane, 2-methyltetrahydrofurane, diglyme, butyl diglyme, water, methanol, ethanol, isopropanol, and mixtures thereof. The base can be selected from aqueous ammonia or sodium hydroxyde (especially aqueous sodium hydroxyde) or potassium hydroxyde (especially aqueous potassium hydroxyde).The reaction temperature is below the boiling point of the solvent used, preferably between 25 and 60°C. The reacting with hydrogen can be carried out in presence of a hydrogenation catalyst. The hydrogenation catalyst can be e.g. a nickel containing hydrogenation catalyst, e.g. Raney nickel. The reaction can be carried out at ambient pressure or at a pressure higher than ambient pressure. The reaction can be carried out e.g. at a pressure between 101 kPa and 400 kPa (4 bar), especially at a pressure between 101 kPa and 400 kPa (4 bar) H₂. The reaction mixture obtained after having reacted compound of formula (II) with hydrogen can be filtrated for removing solids (e.g. catalyst). Solvent can be removed from the filtrated reaction mixture, e.g. by evaporation. The crude product can than be purified by acid/base extraction. Acid/base extraction can be carried out by mixing the crude product with organic solvent (e.g. toluene) and water, adjusting pH to below 2 with aqueous acid (e.g. aqueous HCI), followed by separating the aqueous phase, adding organic solvent (e.g. an ether, such as tert-butyl methyl ether) to aqueous phase and adjusting pH to above 10 with aqueous base (e.g. NaOH), separating the organic phase, and removing solvent from the organic phase. The residue obtained after removal of solvent is or comprises or consists essentially of compound of formula (I).

Furthermore, the compound of formula (I) (e.g. as crude reaction product) can be reacted with acid (e.g. HCI) to prepare a salt of (e.g. HCI salt of) compound of formula (I).

A fourth process step (referred to hereinafter also as step (D.)), is or comprises optionally reacting compound of formula (I) (which can be optionally dissolved or suspended in solvent) 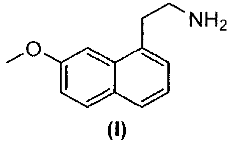 with halogenic acid (especially HCl) to prepare a halogenic acid salt (especially HCI salt) of compound of formula (I), and
reacting the compound of formula (I) (which can be optionally dissolved or suspended in solvent) with acetanhydride (optionally in presence of a base) to obtain agomelatine of formula (A), especially at a temperature in the range 20-25°C, e.g. for 2 to 3 hours, or reacting the optionally prepared halogenic acid salt (especially HCl salt) of compound of formula (I) (which can be optionally dissolved or suspended in solvent) with acetanhydride (optionally in presence of a base), especially at a temperature in the range 20-25°C, e.g. for 2 to 3 hours,
to obtain agomelatine of formula (A).

The compound of formula (I) (e.g. obtained after the third reaction step (C.) ) can be reacted with acid (e.g. HCl) to prepare a salt of (e.g. HCl salt of) compound of formula (I). For example, compound of formula (I) can be dissolved in a solvent, e.g. an organic solvent, and can then be reacted with a solution comprising acid (e.g. HCl) to obtain the salt of (e.g. HCl salt of) compound of formula (I).

In particular, compound of formula (I) (e.g. obtained after the third process step (C.) after removal of solvent)) can be mixed with organic solvent (e.g. ethanol). An acid (e.g. HCl) dissolved in solvent (e.g. in ether, such as tert-butyl methyl ether) can be added to the so obtained mixture to obtain the salt of (e.g. HCl salt of) compound of formula (I). Salt of (e.g. HCl salt of) compound of formula (I) can precipitate and can then be separated by filtration, optionally washed with organic solvent (e.g. ether, such as tert-butyl methyl ether), and dried.

The reacting of compound of formula (I) or of a halogenic acid salt thereof (e.g. HCl salt thereof) with acetanhydride can be carried out in solvent (e.g. solvent as indicated in this paragraph below). The solvent (which can be used for reacting of compound of formula (I) or the halogenic acid salt thereof), especially inert solvent, may be selected from the group consisting of TBME (tert-butyl methyl ether), ethyl acetate, isopropyl acetate, toluene, methanol, ethanol and isopropanol, THF (tetrahydrofuran), 2-MeTHF (2-methyl-tetrahydrofuran), and mixtures thereof. The base (which can be optionally present when reacting of compound of formula (I) or the halogenic acid salt thereof) may be selected from the group consisting of sodium acetate, potassium acetate and ammonium acetate, and mixtures thereof.

In particular, in the process for the preparation of polymorph form X of agomelatine of the present invention, the providing of agomelatine of formula (A) can be or can comprise: preparing agomelatine, said preparing agomelatine of formula (A) comprising: providing compound of formula (I), optionally reacting compound of formula (I) with halogenic acid to prepare a halogenic acid salt of compound of formula (I), and
reacting the compound of formula (I) with acetanhydride to obtain agomelatine of formula (A), or reacting the optionally prepared halogenic acid salt of compound of formula (I) with acetanhydride to obtain agomelatine of formula (A).

The providing of compound of formula (I) can comprise providing a compound of formula (II) and reacting the compound of formula (II) with hydrogen to obtain the compound of formula (I).

The providing of compound of formula (II) can comprise: providing one or more of: compound of formula (III) and compound of formula (V),
subjecting one or more of compound of: formula (III) and compound of formula (V) to an aromatization reaction to obtain a compound of formula (II).

The providing of one or more of: compound of formula (III) and compound of formula (V) can comprise: providing a compound of formula (IV), and reacting compound of formula (IV) with dialkyl cyanomethylphosphonate, preferably diethyl cyanomethylphosphonate, to obtain one or more of: compound of formula (III) and compound of formula (V).

Agomelatine can be also prepared in a one pot reaction starting from compound of formula (II), especially by carrying out reaction steps (C.) and (D.), as described herein, without isolation of products after step (C.)

The compound 7-methoxy-1-tetralone is a known compound and is also commercially available. Table 2 below provides an overview of process steps which may be used for preparing 7-methoxy-1-tetralone.

**Table 2**

| |
|---|
| Friedel-Crafts acylation |
| 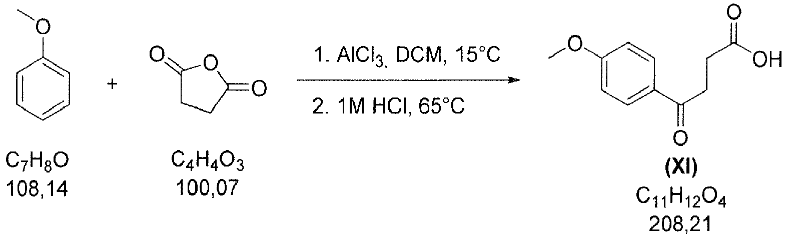 |
| Reduction of ketone |
| 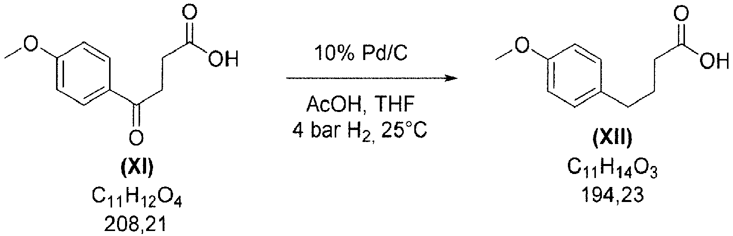 |
| Intramolecular cyclization |
| 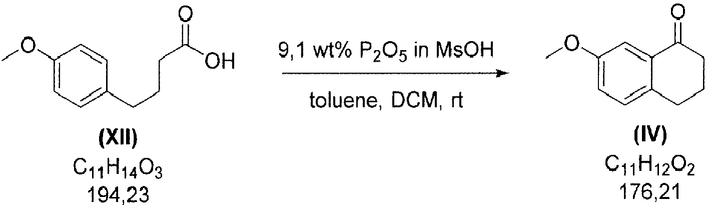 |

Agomelatine polymorph form X (which can be obtainable by the process of the present invention) can be characterized by a DSC (differential scanning calorimetry) thermogram showing an (endothermic) peak with peak T onset at 95-97 °C, optionally with melting enthalpy of 100 to 130 J/g, which DSC thermogram can optionally further show an (endothermic) peak with peak T onset at 107 to 109°C, optionally with melting enthalpy of less than 60 J/g. Melting enthalpy can be determined by DSC method, especially at a heating rate of 1°C /minute.

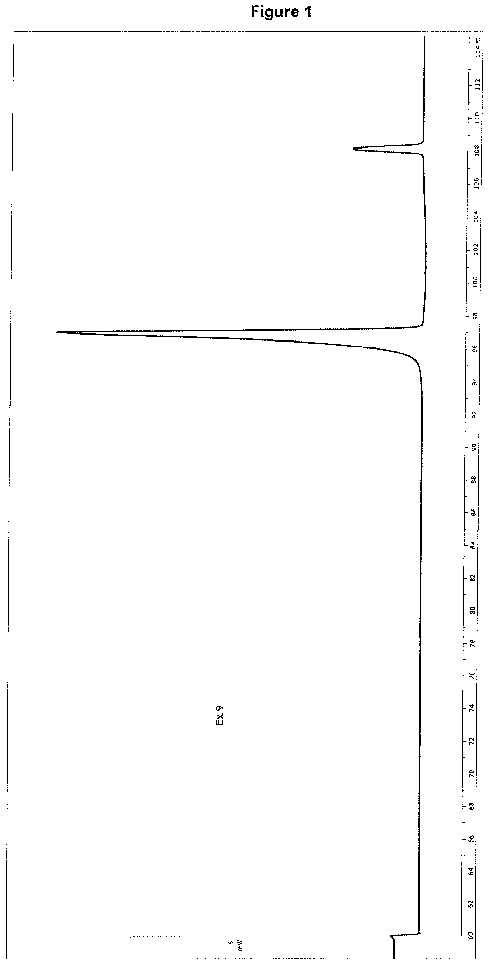

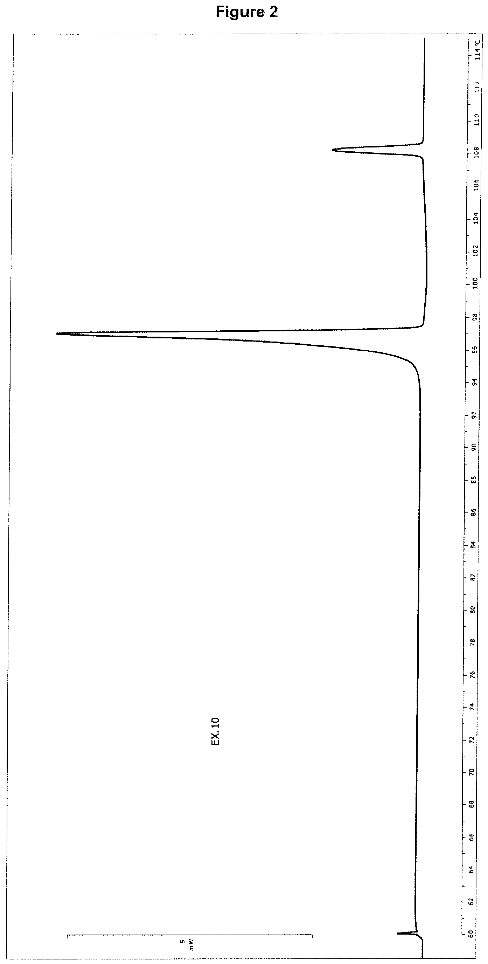

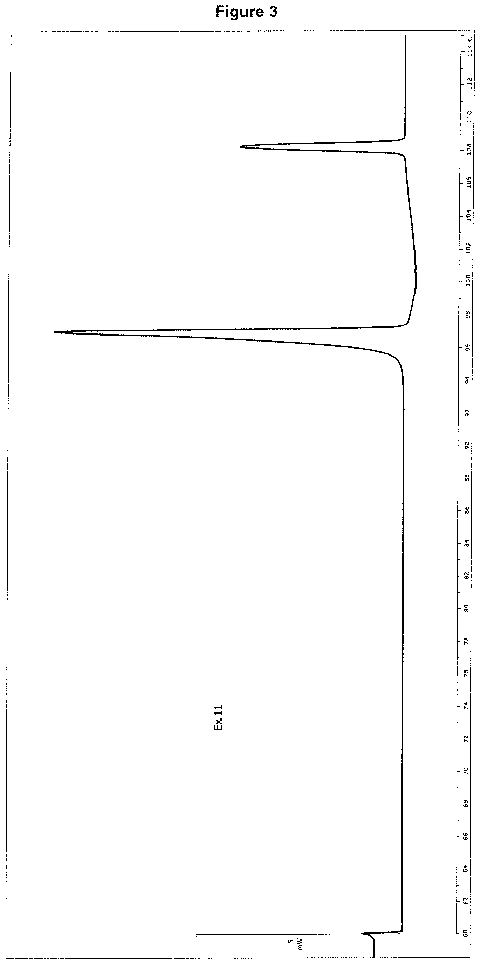

Preferably, agomelatine polymorphic form X (which can be obtainable by the process of the present invention) can be characterized by a DSC thermogram showing an (endothermic) peak with peak T onset at 96.3 °C, optionally with a melting enthalpy of 116 J/g, which DSC thermogram can optionally further show an (endothermic) peak with peak T onset at 107.9°C, optionally with a melting enthalpy of less than 60 J/g. More preferably, agomelatine polymorphic form X (which can be obtainable by the process of the present invention) can be characterized by a DSC thermogram substantially as depicted in Fig. 1 or 2 or 3 , especially as depicted in Fig. 1 or 2 or 3 in the range 90°C to 112°C, in particular in the range 90°C to 103°C. (DSC thermogram can be determined by DSC method, especially at a heating rate of 1°C /minute.)

Agomelatine polymorphic form X can be agomelatine in the polymorphic form disclosed in EP 2 474 522 A1. Table 3 summarizes data disclosed in EP 2 474 522 A1 for polymorphic form X:

**Table 3**

| **Analytic data for Agomelatine polymorphic form X (according to** EP 2 474 522 A1**)** | | |
|---|---|---|
| | | |
| 1. X-ray powder diffraction: | 2 θ | I/Iₒ |
| Instruments: Rigaku, Japan, D/MAX 2500 X-ray diffractometer | 12. 84 | 25 |
| Target: Cu-Kα radiation(λ= 1.5405), 2θ=2-40°C | 13. 84 | 23 |
| Step angle : 0.04°C | 16. 14 | 37 |
| Calculated time: 0.5 s | 18. 56 | 100 |
| Tube voltage: 40KV | 19. 12 | 27 |
| Tube current: 100mA | | |
| Scanning speed: 8°C/min | 20. 86 | 75 |
| Filter: Graphite monochromator | | |
| Error of 2θ value: 2θ value±0.10 | 21. 20 | 30 |
| | 23. 84 | 64 |
| | | |
| Infrared spectroscopy (IR): | | |
| Instruments: Nicolet, US, MAGNA-560 Fourier transform infrared spectrometer. | | |
| The wave numbers of IR spectrum for the ag omelatine crystal using potassium bromide for grinding are as follows: characteristic absorption peaks at 3234, 3060, 2940, 1638, 1511, 1436, 1249, 1215, 1184, 1032, 908, 828, 755, 588 cm⁻¹. | | |

In particular, a crystal of agomelatine polymorphic form X can be characterized by one or more of: X-ray powder diffraction pattern in the 2Θ using Cu-Kα radiation having characteristic diffraction peaks at 12.84, 13.84, 16.14, 18.56, 19.12, 20.86, 21.20, 23.84, IR absorption pattern having characteristic absorption peaks at 3234, 3060, 2940, 1638, 1511, 1436, 1249, 1215, 1184, 1032, 908, 828, 755, 588 cm⁻¹.

According to a further aspect, there is provided a Polymorph form X of agomelatine of formula (A) obtainable according to the process for the preparation of polymorph form X of agomelatine of the present invention, as described herein.

According to a further aspect, there is provided a polymorph form X of agomelatine characterised by Δ H (J/g) value for form II of below 60, preferably below 30, more preferably below 20, even more preferably below 10, as determined by DSC method, especially at heating rate 1°C/minute. The DSC thermogram can be measured when agomelatine polymorph form X is subjected to heating (e.g. starting from a temperature of 20°C) at a heating rate of 1°C /minute by a differential scanning calorimeter.

For example, polymorph form X of agomelatine can be characterised by Δ H (J/g) value for form II in the range of 1 to below 60, as determined by DSC method (in particular at a heating rate of 1°C /minute). Preferably, said agomelatine polymorph form X can be further characterized by a DSC thermogram showing an (endothermic) peak with peak T onset at 95-97 °C (preferably at 96.3 °C), optionally with a melting enthalpy of 100 to 130 J/g (preferably 116 J/g), which DSC thermogram can still further show a peak with peak T onset at 107 to 109°C (preferably at 107.9°C), optionally with a melting enthalpy (Δ H) of less than 60J/g; e.g., said agomelatine polymorphic form X can be characterized by a DSC thermogram substantially as depicted in Fig. 1 or 2 or 3. Said polymorph form X of agomelatine can be obtainable according to the process for the preparation of polymorph form X of agomelatine of the present invention, as described herein.

ΔH value can be determined by integration of the area under the (endothermic) curve, especially by computer program. Δ H (J/g) value for form II (also referred to herein as "Δ H (J/g) form II" or as ΔₘH(form II)) can indicate the melting enthalpy of Form II, especially determined by DSC method. Values determinable by DSC method, such as melting enthalpies, and in particular Δ H (J/g) value, e.g. for form II, can be determined by DSC method at a heating rate of 1°C/minute; the DSC thermogram can be measured when agomelatine polymorph form X is subjected to heating (e.g. starting from a temperature of 20°C) at a heating rate of 1°C /minute by a differential scanning calorimeter. Especially, the DSC thermogram can be measured when 3 mg of agomelatine polymorph form X is heated (e.g. from 20°C to 115°C) at a heating rate of 1°C /minute by a differential scanning calorimeter.

Optionally, this polymorph form X of agomelatine is obtainable according to the process for the preparation of polymorph form X of agomelatine of the present invention, as disclosed herein.

Three thermal effects are usually observed on the recorded thermograms (according to DSC technique) of agomelatine form X. The first one appears as endothermic peak in temperature range 90 - 100 °C, corresponding to melting of agomelatine form X. The second one appears as exothermic peak just after the first one and corresponds to crystallization of melt agomelatine to form II. The third appears as endothermic peak in temperature range 105 - 110 °C, corresponding to melting of formed agomelatine form II. Both endothermic peaks are integrated against a baseline.

Stability of agometaline form X against phase transition to agomelatine form II is evaluated based on enthalpies of melting of both endothermic peaks (Form II, Form X) determined according to DSC technique. In an embodiment, a DSC (Differential Scanning Calorimetry) thermogram is recorded (e.g. according to the method as described herein below in the paragraph "METHODS") for a sample of agometaline form X of interest. On the basis of the DSC (Differential Scanning Calorimetry) thermogram recorded, melting enthalpy of Form II and melting enthalpy of Form X is determined. Generally, thermograms (according to DSC technique) of more stable samples reveal lower values of ΔₘH(form II). Particularly appropriate value ranges of ΔₘH(form II) for agomelatine form X to be considered as stable are lower than 60 J/g, preferably lower than 40 J/g, preferably lower than 20 J/g (and can be determined by DSC method, especially at a heating rate of 1°C /minute).

Agomelatine polymorphic form II can be agomelatine in the polymorphic form disclosed in EP 1 564 202 B1, especially claim 16 thereof. In particular, a crystal of agomelatine polymorphic form II can be characterized by one or more of the following data, obtained from a powder diagram, in particular obtained from
the powder diagram produced using a Bruker AXS D8
high-resolution diffractometer having an angular field 2θ of 3°-90°, a step of 0.01° and 30 s per step :
- monoclinic crystal lattice,
- lattice parameters : a = 20.0903 Å, b = 9.3194 Å, c = 15.4796 Å, β = 108.667°
- space group : P2₁/n
- number of molecules in the lattice : 8
- lattice volume : V_{lattice} = 2746.742 Å³
- density : d = 1.13 g/cm³.

According to one aspect, there is provided a method for the preparation of agomelatine (A) 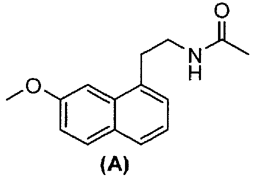 said method comprising:
(A.) reacting a compound of formula (IV) 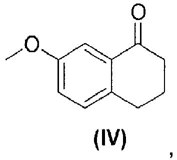 with dialkyl cyanomethylphosphonate, preferably diethyl cyanomethylphosphonate, to obtain one or more of: compound of formula (III) and compound of formula (V) 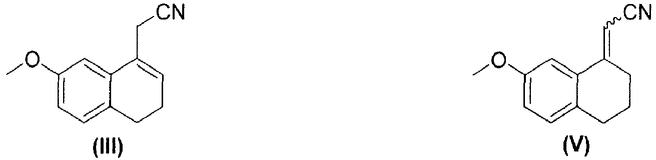
(B.) subjecting the one or more of: compound of formula (III) and compound of formula (V) to an aromatization reaction (preferably by contacting the one or more of: compound of formula (III) and compound of formula (V) with a hydrogenation catalyst) to obtain a compound of formula (II) 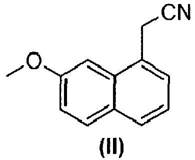
(C.) reacting the compound of formula (II) with hydrogen to obtain a compound of formula (I) 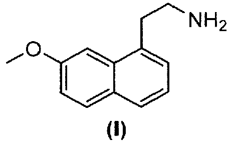
(D.) optionally reacting the compound of formula (I) with halogenic acid to prepare a halogenic acid salt of compound of formula (I), and
   reacting the compound of formula (I) with acetanhydride to obtain agomelatine of formula (A), or
   reacting the optionally prepared halogenic acid salt of compound of formula (I) with acetanhydride to obtain agomelatine of formula (A),
(E.) optionally crystallizing polymorph form X of agomelatine in the presence of acid. The crystallizing polymorph form X of agomelatine in the presence of acid can be in particular carried out according to process for the preparation of polymorph form X of agomelatine of formula (A) as defined herein.

If not explicitly mentioned to the contrary herein, the term room temperature can have the meaning of 20°C.

### METHODS

### DSC analysis:

Samples of agomelatine form X are characterized by differential scanning calorimetry (DSC) using a Mettler Toledo DSC 1 STAR ^{e} System differential scanning calorimeter according to following method:
Approximately 3 mg of a sample is accurately weighted in a 40 µl aluminium pan. The pan is tightly sealed with an aluminium lid, which is pierced before the sample is inserted into the oven. A thermogram is recorded in nitrogen atmosphere with flow rate 40 ml/min within temperature range 20 - 115 °C at heating rate 1 °C/min.

The following examples illustrate the invention and are not intended to restrict the scope of the invention in any way.

### Examples

### Reference Example 1

### Crystallization of agomelatine form X in water

N-(2-(7-methoxynaphthalen-1-yl)ethyl)acetamide (60 g) was dissolved in DMF (300 mL). Solution was added under stirring dropwise in 10L reactor comprising water (6 L) and 0.8 g of seeds of agomelatine form X at 25°C (room temperature). Precipitate started to form. Suspension was filtered, precipitate washed with water and dried to collect N-(2-(7-methoxynaphthalen-1-yl)ethyl)acetamide form X (49,6 g, yield 83%).

### Example 1

### Crystallization of agomelatine form X in water with tartaric acid (0.1 eq)

N-(2-(7-methoxynaphthalen-1-yl)ethyl)acetamide (20 g) was dissolved in DMF (100 mL). Solution was added under stirring dropwise in 3L reactor comprising water (2 L) and tartaric acid (1,2 g) and 0.3 g of seeds of agomelatine form X at 25°C (room temperature). Precipitate started to form. Suspension was filtered, precipitate washed with water and dried to collect N-(2-(7-methoxynaphthalen-1-yl)ethyl)acetamide form X (16.0 g, yield 80%).

### Example 2

### Crystallization of agomelatine form X in water with tartaric acid (1.0 eg)

N-(2-(7-methoxynaphthalen-1-yl)ethyl)acetamide (20 g) was dissolved in DMF (100 mL). Solution was added under stirring dropwise in 3L reactor comprising water (2 L) and tartaric acid (12 g) and 0.3 g of seeds of agomelatine form X at 25°C (room temperature). Precipitate started to form. Suspension was filtered, precipitate washed with water and dried to collect N-(2-(7-methoxynaphthalen-1-yl)ethyl)acetamide form X (16.0 g, yield 80%).

### Example 3

### Crystallization of agomelatine form X in water with acetic acid (0.1 eq)

N-(2-(7-methoxynaphthalen-1-yl)ethyl)acetamide (20 g) was dissolved in DMF (100 mL). Solution was added under stirring dropwise in 3L reactor comprising water (2 L), 0.3g of seeds of agomelatine form X and acetic acid (0.48 mL) at 25°C (room temperature). Precipitate started to form. Suspension was filtered, precipitate washed with water and dried to collect N-(2-(7-methoxynaphthalen-1-yl)ethyl)acetamide form X (16.3 g, yield 81,5%).

### Example 4

### Crystallization of agomelatine form X in water with acetic acid (0.05 eq)

2000 mL of water and 0.25 mL of acetic acid was charged to reactor at 25 °C and ambient pressure. The batch was seeded with 0.3 g of agomelatine form X. In a 250 ml flask was added 20,0 g of agomelatine and 100 ml *N*,*N*-Dimethylformamide and mixed until a clear solution was obtained. Solution was filtered through GF/B filter and added to reactor in 15 seconds at 150 rpm. The mixture was stirred for 25 min, filtered and isolated cake was washed with 400 mL of water. Yield = 85 %.

### Example 5

### Crystallization of agomelatine form X in water with acetic acid (1.0 eq)

2000 mL of water and 4.8 mL of acetic acid was charged to reactor at 25 °C and ambient pressure. The batch was seeded with 0.3 g of agomelatine form X. In a 250 ml flask was added 20.0 g of agomelatine and 100 ml *N*,*N*-Dimethylformamide and mixed until a clear solution was obtained. Solution was filtered through GF/B filter and added to reactor in 15 seconds at 150 rpm. The mixture was stirred for 25 min, filtered and isolated cake was washed with 400 mL of water. Yield = 85 %.

### Example 6

### Crystallization of agomelatine form X in water with HCl (0.1 eq.)

2000 mL of water and 0,25 mL of hydrochloric acid (37 %) was charged to reactor at 25 °C and ambient pressure. The batch was seeded with 0,3 g of agomelatine form X. In a 250 ml flask was added 20,0 g of agomelatine and 100 ml *N*,*N*-Dimethylformamide and mixed until a clear solution was obtained. Solution was filtered through GF/B filter and added to reactor in 15 seconds at 150 rpm. The mixture was stirred for 25 min, filtered and isolated cake was washed with 400 mL of water. Yield = 83 %.

### Example 7

### Crystallization of agomelatine form X in water with HCl (0.05 eq.)

2000 mL of water and 0.125 mL of hydrochloric acid (37 %) was charged to reactor at 25 °C and ambient pressure. The batch was seeded with 0,3 g of agomelatine form X. In a 250 ml flask was added 20,0 g of agomelatine and 100 ml *N*,*N*-Dimethylformamide and mixed until a clear solution was obtained. Solution was filtered through GF/B filter and added to reactor in 15 seconds at 150 rpm. The mixture was stirred for 25 min, filtered and isolated cake was washed with 400 mL of water. Yield = 83 %.

### Example 8

### Crystallization of agomelatine form X in water with Na acetate (1,0 eq.)

2000 mL of water and 6,7 g of sodium acetate was charged to reactor at 25 °C and ambient pressure. The batch was seeded with 0,3 g of agomelatine form X.

In a 250 ml flask was added 20,0 g of agomelatine and 100 ml *N*,*N*-Dimethylformamide and stirred until a clear solution was obtained. Solution was filtered through GF/B filter and added to reactor comprising water in 15 seconds under stirring at 150 rpm. The mixture was stirred for 25 min, filtered and isolated cake was washed with 400 mL of water. Yield = 81 %.

### Example 9

### Crystallization of agomelatine form X in water with acetic acid (0.05 eq)

2000 mL of water and 0,25 mL of acetic acid was charged to reactor at 25 °C and ambient pressure. The batch was seeded with 0.3 g of agomelatine form X. In a 250 ml flask was added 20.0 g of agomelatine and 100 ml *N*,*N*-Dimethylformamide and mixed until a clear solution was obtained. Solution was filtered through GF/B filter and added to reactor in 15 seconds at 150 rpm. The mixture was stirred for 25 min, filtered and isolated cake was washed with 400 mL of water. Yield = 82 %.

### Example 10

### Crystallization of agomelatine form X in water with acetic acid (0.1 eq)

2000 mL of water and 0,50 mL of acetic acid was charged to reactor at 25 °C and ambient pressure. The batch was seeded with 0.3 g of agomelatine form X. In a 250 ml flask was added 20.0 g of agomelatine and 100 ml *N*,*N*-Dimethylformamide and mixed until a clear solution was obtained. Solution was filtered through GF/B filter and added to reactor in 15 seconds at 150 rpm. The mixture was stirred for 25 min, filtered and isolated cake was washed with 400 mL of water. Yield = 81 %.

### Example 11

### Crystallization of agomelatine form X in water with acetic acid (0.05 eq)

5500 mL of water and 0,68 mL of acetic acid was charged to reactor at 25 °C and ambient pressure. The batch was seeded with 0.8 g of agomelatine form X. In a 500 ml flask was added 55.0 g of agomelatine and 275ml *N*,*N*-Dimethylformamide and mixed until a clear solution was obtained. Solution was filtered through GF/B filter and added to reactor in 30 seconds at 180 rpm. The mixture was stirred for 25 min, filtered and isolated cake was washed with 400 mL of water. Yield = 83 %.

The table below shows that an addition of different substances can influence stability of the polymorphic form X which is reflected by enthalpy for Form II as determined by DSC. The inventors have found that a particularly advantageous stability of polymorphic form X is achieved for a batch having ΔH for Form II ≤ 60 J / g. In a preferred embodiment, the enthalpy is as low as possible, which would in turn mean that the conversion to form II is slower. In order to confirm this criterion, the respective samples were also subjected to an accelerated stability test. With the addition of acetic acid during the process for the preparation of polymorph form X of agomelatine, the lowest values of ΔH for form II were obtained at different concentrations; therefore acetic acid is a particularly preferred additive advantageously influencing the stability of crystalline agomelatine polymorphic form X.

**Table 4**

| **Example** | **Addition to water** | **Physical stability (1 day/80 °C)** | **Physical stability (3 days/80 °C)** | **DSC analysis Δ H (J/g) form II** |
|---|---|---|---|---|
| **Reference Ex.1** | *l* | X + 5-10 % II | X + 50% II | 91,3 |
| **Ex1** | 0,1 eq. tartaric acid seeding | *l* | *l* | 2,2 |
| **Ex2** | 1,0 eq. tartaric acid | / | *l* | 19,1 |
| **Ex3** | 0,1 eq. acetic acid (0,24 mg/mL) seeding | *l* | X+traces of form II | **1,7** |
| **Ex4** | 0,05 eq. acetic acid (0,12 mg/mL) | *l* | X+traces of form II | **2,7** |
| **Ex5** | 1,0 eq. acetic acid (2,40 mg/mL) | *l* | *l* | **1,3** |
| **Ex6** | 0,1 eq. HCl seeding | *l* | *l* | 6,5 |
| **Ex7** | 0,05 eq. HCl | *l* | *l* | 11 |
| **Ex8** | 1,0 eq. Na acetate | *l* | *l* | 31,8 |
| **Ex 9** | 0.05 eq acetic acid seeding | *l* | / | 13 |
| **Ex 10** | 0.1 eq acetic acid seeding | *l* | *l* | 15.9 |
| **Ex 11** | 0.05 eq acetic acid seeding | X | X+5% form II | 37.6 |

Reproducing prior art processes for obtaining form X did not provide stable polymorphic form X. Under stability testing conditions, the polymorph obtained by reproducing prior art processes converts partly or completely to the thermodynamically most stable polymorphic form of agomelatine form II, immediately after production or under stability testing conditions. The results of these prior art processes were not reproducible and robust; often under the same reaction conditions products having different stability were obtained.

The process of the present invention is a robust and reliable process with high reproducibility, especially in terms of obtaining stable polymorphic form X as determined by Δ H (J/g) value.

### Example 12: Synthesis of compound of formula (XI)

### Synthesis of 4-(4-methoxyphenyl)-4-oxobutanoic acid

A 5L reactor was charged with dichloromethane (1,17 L) and aluminium trichloride (454,5 g) and cooled to 5°C. Anisole (342 mL) was added dropwise to the cooled suspension and after completion, reaction mixture was mixed for 1,5 h at 5°C. Then succinic anhydride (262,5 g) was added portionwise in 3 h at 5°C. Dark reddish reaction mixture was allowed to stir for 18 h at 5°C. Cooled reaction mixture was added dropwise to 1M HCl solution (6,5 L) heated at 60°C, and in the same time dichloromethane was distilled off. White precipitate starts to form. After the addition the rest of dichlormethane is distilled off under reduced pressure. White suspension is then cooled to 5 °C, collected upon vacuum filtration and washed with toluene. The product (484 g, yield 88 %) was isolated as a white solid.

### Example 13: Synthesis of compound of formula (XII)

### Synthesis of 4-(4-methoxyphenyl)butanoic acid

4-(4-methoxyphenyl)-4-oxobutanoic acid (650 g), tetrahydrofuran (2,6 L), acetic acid (2,6L) and 10 % Pd/C (13 g) is charged in 10L hydrogenator. Reaction mixture is stirred at 30 °C and 5 bar H₂. Reaction is monitored with HPLC and after completion, reaction mixture is filtered. Further toluene (3,13 L) is added, extracted with water (3,13 L) and solvent is evaporated. Product is obtained as white solid (570 g, yield 94 %)

### Example 14: Synthesis of compound of formula (IV)

### Synthesis of 7-methoxy-1-tetralone

To 4-(4-methoxyphenyl)butanoic acid (285 g) toluen (285 mL) and dichloromethane (2,425 L) is added and cooled to 15°C. Slowly Eaton's reagent (1,54 L; Eaton's reagent being: 9,1 wt% P₂O₅ in methanesulfonic acid) is added, and colour is changed to dark yellowish brown. Reaction mixture is then heated to room temperature and monitored with HPLC. When reaction is completed it is quenched in mixture of *tert*-butyl methyl ether (3,04 L) and water (3,04 L) cooled at 2°C. Organic phase was separeted and washed with saturated water solution of sodium bicarbonate (3,04 L). Solvent is evaporated and further crystallization in 80% ethanol afforded 7-methoxy-1-tetralone as yellow crystals (267g, yield 91%).

### Example 15: HWE (Horner Wadsworth Emmons) REACTION

### Synthesis of 2-(7-methoxy-3,4-dihydronaphthalen-1(2H)-ylidene)acetonitrile (Synthesis of one or more of: compound of formula (III) and compound of formula (V), especially synthesis of a mixture comprising cis-form of compound of formula (V), trans-form of compound of formula (V), and compound of formula (III))

### EXAMPLE 15-1

In a 1 L three necked reactor, tetrahydrofurane (280 mL) and solution of sodium hexamethyl disilazane in tetrahydrofurane (256 mL of 40% solution) were added. The solution is cooled to 0°C and diethyl cyanomethylphosphonate (82.6 mL) is slowly added, maintaining the temperature below 5°C. The reaction mixture is warmed to room temperature and stirred for 1 hour.

Thereafter, the solution of 7-methoxy-1-tetralone (60 g) in tetrahydrofurane (100 mL) is added dropwise, and the reaction mixture stirred at room temperature for another 4 hours. Once the reaction is finished, 10% solution of acetic acid in water (500 mL) is added maintaining the temperature below 35°C. The mixture is extracted with toluene (300 mL). Layers are separated, and organic layer is washed with water (500 mL). The solvent is evaporated and the residue is used in the next step without further purification.

### EXAMPLE 15-2.

In a 1 L reactor, 7-methoxy-1-tetralone (50 g), potassium carbonate (117.6 g), diethyl cyanomethylphosphonate (69 mL) and isopropanole (500 mL) were added. The solution is refluxed until the complete conversion to products.

Once the reaction is finished, is cooled to room temperature and water (500 mL) and toluene (500 mL) were added. Layers were separated, and organic layer is washed with water (500 mL). The solvent is evaporated and the residue is used in the next step without further purification. The composition of product according to HPLC: 22.0% compound (III), 24.6% cis-form of compound (V) and 52.3% trans-form of compound (V).

### EXAMPLE 15-3.

In a 200 mL reactor, 7-methoxy-1-tetralone (10 g), potassium phosphate tribasic (36.2 g), diethyl cyanomethylphosphonate (13.8 mL) and isopropanole (100 mL) were added. The solution is refluxed until the complete conversion to products.

Once the reaction is finished, is cooled to room temperature and water (100 mL) and toluene (100 mL) were added. Layers were separated, and organic layer is washed with water (50 mL). The solvent is evaporated and the residue is used in the next step without further purification. The composition of product according to HPLC: 28.0% compound (III), 11.0% cis-form of compound (V) and 55.0% trans-form of compound (V)

### EXAMPLE 15-4.

In a 200 mL reactor, 7-methoxy-1-tetralone (10 g), caesium carbonate (55.5 g), diethyl cyanomethylphosphonate (13.8 mL) and isopropanole (100 mL) were added. The solution is refluxed until the complete conversion to products.

Once the reaction is finished, is cooled to room temperature and water (100 mL) and toluene (100 mL) were added. Layers were separated, and organic layer is washed with water (50 mL). The solvent is evaporated and the residue is used in the next step without further purification. The composition of product according to HPLC: 29.4% compound (III), 3.8% cis-form of compound (V) and 60.4% trans-form of compound (V)

### Example 16: AROMATIZATION

### Synthesis of 2-(7-methoxynaphthalen-1-yl)acetonitrile (compound of formula (II))

### EXAMPLE 16-1.

Diglyme (1,01 L) and 5 % Pd/C (13,4 g) is charged in 2L reactor, heated to 140°C and than 1/3 volume of diglyme is evaporated to remove water. Suspension is then cooled to 100°C, purged with nitrogen, then diglyme (337 mL), 4-nitrotoluene (16,6 g) and mixture of compound of formula (III), cis-form of compound of formula (V), trans-form of compound of formula (V) from previous step (133,6 g) is added and reaction mixture is heated to 140°C for 19h. After reaction is finished it is cooled, filtered and diglyme is evaporated. To concentrate is added xylene (1 L) and water (750 mL), mixed and phases are separated. Organic phase is evaporated and the product was crystallized to obtain off white solid (107 g, yield 84%).

### EXAMPLE 16-2.

In reactor a mixture of compound of formula (III), cis-form of compound of formula (V), trans-form of compound of formula (V) (100 g) from previous step is charged than 4-nitrotoluene (13,5 g) and 10% Pd/C (8,8 g) is added. Mixture is heated to 130 °C for 19h. Reaction mixture is then cooled, tert-butyl methyl ether is added (1L) and filtered to obtain 83,4% product according to HPLC.

### EXAMPLE 16-3.

In 10L reactor a mixture of compound of formula (III), cis-form of compound of formula (V), trans-form of compound of formula (V)_(170g) is charged along with 5% Pd/C (31,2 g) 2-methyl tetrahydrofuran (2,5 L) and cyclohexane (275 mL). Reactor is tightly sealed and inertizide with nitrogen. Reaction mixture is heated to 130°C for 4 days and after crystallization in 2-propanol obtained product 97,7 % purity according to HPLC.

### Example 17: Synthesis of compound of formula (I)

### Synthesis of 2-(7-methoxynaphthalen-1-yl)ethan-1-amine hydrochloride

In a 10L reactor compound of formula II (230 g), methanol (3,7 L), NaOH solution ((123,3) g NaOH is diluted in 460mL water) and Raney Nickel (41,193 g) is charged. Reaction mixture is stirred at 30°C and 4 bar H₂ and monitored with HPLC. After reaction is done, it is filtered and solvent is evaporated. To concentrate is added toluene (4,25 L) and water (2,13 L), mixed and separated. To organic phase water is added (2,13 L), pH is adjusted to 2 with 37 % HCI, and phases are separated. To water phase *tert*-butyl methyl ether (2,13 L) is added and pH is adjusted to 10 with 40 % NaOH. Further organic phase is concentrated, ethanol (230 mL) is added, heated to 50°C then 4 M HCl in tert-butyl methyl ether (355 mL) and *tert-*butyl methyl ether (920 mL). White precipitate started to obtain. Suspension is cooled to room temperature, filtered, washed with *tert*-butyl methyl ether and dried. Product is white solid (256,2, yield 92%).

### Example 18: Synthesis of compound of formula (A)- one pot synthesis from compound of formula II

In 10L reactor 2-(7-methoxynaphthalen-1-yl)acetonitrile (750g), methanol (6,3L), NaOH solution (228g NaOH is diluted in 750mL water) and 134g of Raney-Nickel is charged. Reaction mixture is stired at 30°C and 4 bar H₂ and monitored with HPLC. After reaction is done, it is cooled to 15°C and acetic anhydride (750mL) is added and warmed to 25°C. Reaction is monitored with HPLC. Once the reaction is finished it is filtered, solvent evaporated, than ethyl acetate (5L) is added and organic phase is extracted with water (5L). Solvent is evaporated to obtain agomelatine of 96,4% HPLC purity.

### Example 19: Synthesis of compound of formula (A)

### Synthesis of agomelatine

2-(7-methoxynaphthalen-1-yl)ethan-1-amine hydrochloride (245 g), isopropyl acetate (2,45 L) and sodium acetate trihydrate (140,3 g) is charged in 5 L reactor. To white suspension acetic anhydride (107 mL) is added dropwise. Reaction is monitored with TLC and after completition is extracted with NaHCO₃ solution and water. Solvent is evaporated. (250g, yield 99%).

### Example 20

### DSC analysis:Samples of agomelatine form X are characterized by differential scanning calorimetry (DSC)

Approximately 3 mg of a sample is accurately weighted in a 40 µl aluminium pan. The pan is tightly sealed with an aluminium lid, which is pierced before the sample is inserted into the oven. A thermogram is recorded in nitrogen atmosphere with flow rate 40 ml/min within temperature range 20 - 115 °C with heating rate 1 °C/min.

Three thermal effects are usually observed on the recorded thermograms of agomelatine form X. The first one appears as endothermic peak in temperature range 90 - 100 °C, corresponding to melting of agomelatine form X. The second one appears as exothermic peak just after the first one and corresponds to crystallization of melt agomelatine to form II. The third appears as endothermic peak in temperature range 105 - 110 °C, corresponding to melting of formed agomelatine form II. Both endothermic peaks are integrated against a baseline.

Stability of agomelatine form X against phase transition to agomelatine form II is evaluated based on enthalpies of melting of both endothermic peaks. Generally, thermograms of more stable samples reveal lower values of ΔₘH(form II). Appropriate value of ΔₘH(form II) for agomelatine form X to be considered as stable is lower than 60 J/g, preferably lower than 40 J/g, preferably lower than 20 J/g.

### Example 21

### Crystallization of agomelatine form X in water with tartaric acid (0.1 eq)

N-(2-(7-methoxynaphthalen-1-yl)ethyl)acetamide (20 g) was dissolved in DMF (100 mL). Solution was added under stirring dropwise in 3L reactor comprising water (2 L) and tartaric acid (1,2 g) at 25°C (room temperature). Precipitate started to form. Suspension was filtered, precipitate washed with water and dried to collect N-(2-(7-methoxynaphthalen-1-yl)ethyl)acetamide form X.

### Reference Example 2

### Crystallization of agomelatine form X

3000 mL of water was charged to reactor at 25 °C and ambient pressure. In a 250 ml flask was added 20.0 g of agomelatine and 100 ml *N*,*N*-Dimethylformamide and mixed until a clear solution was obtained. Solution was filtered through GF/B filter and added to reactor in 1 minute at 400 rpm. The mixture was stirred for 1 hour, filtered and isolated cake was washed with 400 mL of water. Yield = 81 %

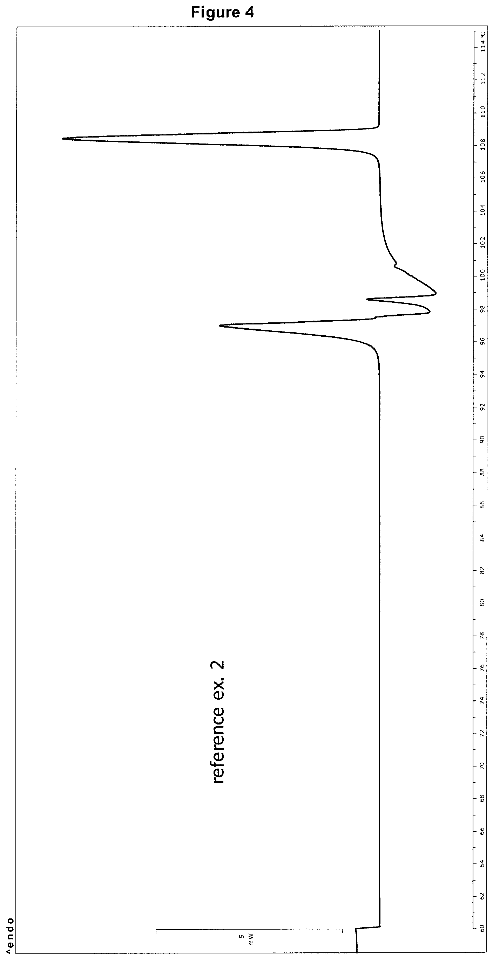

The corresponding product is characterized by Figure 4 below, exhibiting enthalpy for form II of 123.08 J/g.

## Claims

1. Process for the preparation of polymorph form X of agomelatine of formula (A) 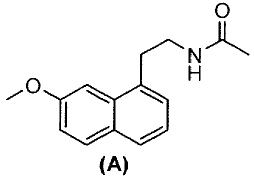 said process comprising:
providing agomelatine of formula (A),
crystallizing agomelatine of formula (A) in the presence of at least one of an acid and a salt thereof,
said crystallizing of agomelatine of formula (A) in the presence of at least one of an acid and a salt thereof comprising:
a) dissolving agomelatine in a first solvent to obtain a solution, said first solvent being or comprising an organic solvent,
b) preparing a mixture comprising
(i) at least one of an acid and a salt thereof, and
(ii) a second solvent,
the at least one of an acid and a salt thereof being dissolved in the second solvent, and
the second solvent being water or a solvent mixture comprising water,
c) adjusting the temperature of the mixture comprising (i) at least one of an acid and a salt thereof and (ii) a second solvent to a temperature in the range from 15°C to 40°C,
d) combining the solution comprising agomelatine with the mixture having a temperature in the range from 15°C to 40°C and comprising i) at least one of an acid and a salt thereof, and ii) a second solvent,
e) isolating crystals of polymorph form X of agomelatine, and
f) optionally drying the isolated crystals of polymorph form X of agomelatine.

2. Process for the preparation of polymorph form X of agomelatine according to claim 1 wherein steps c) and d) are:
c) adjusting the temperature of the mixture comprising (i) at least one of an acid and a salt thereof and (ii) a second solvent to a temperature in the range from 15°C to 40°C, and adding seed crystals of polymorph form X of agomelatine to the mixture,
d) combining the solution comprising agomelatine with the mixture having a temperature in the range from 15°C to 40°C and comprising i) at least one of an acid and a salt thereof, ii) a second solvent, and (iii) seed crystals of polymorph form X of agomelatine.

3. Process for the preparation of polymorph form X of agomelatine according to any one of the preceding claims, wherein in step d) the solution comprising agomelatine has a temperature in the range from 15°C to 40°C, optionally in the range from 17°C to 35°C, further optionally in the range from 20°C to 30°C, still further optionally in the range from 23°C to 27°C, still further optionally in the range from 24-26°C, especially about 25°C.

4. Process for the preparation of polymorph form X of agomelatine according to any one of the preceding claims, wherein the at least one of an acid and a salt thereof is selected from the group consisting of organic acids, inorganic acids, salts of organic acids, salts of inorganic acids, and mixtures thereof,
preferably the at least one of an acid and a salt thereof is selected from compounds comprising one or two or more carboxylic acid moieties, salts of compounds comprising one or two or more carboxylic acid moieties, compounds comprising one or two or more sulfonic acid moieties, salts of compounds comprising one or two or more sulfonic acid moieties, and mixtures thereof.

5. Process for the preparation of polymorph form X of agomelatine according to any one of the preceding claims, wherein the at least one of an acid and a salt thereof is selected from the group consisting of acetic acid, tartaric acid, salts of acetic acid, salts of tartaric acid, hydrochloric acid, and mixtures thereof.

6. Process for the preparation of polymorph form X of agomelatine according to any one of the preceding claims, wherein in step d) both the solution comprising agomelatine and the mixture comprising i) at least one of an acid and a salt thereof, and ii) a second solvent have a temperature in the range from 17°C to 35°C, further optionally in the range from 20°C to 30°C, still further optionally in the range from 23°C to 27°C, still further optionally in the range from 24-26°C, especially about 25°C.

7. Process for the preparation of polymorph form X of agomelatine according to any one of the preceding claims, wherein the first solvent is a solvent selected from the group consisting of dimethylformamide (DMF), toluene, xylene, and mixtures thereof.

8. Process for the preparation of polymorph form X of agomelatine according to any one of the preceding claims, wherein the first solvent is not an organic acid, especially is not an aliphatic acid.

9. Process for the preparation of polymorph form X of agomelatine according to any one of the preceding claims, wherein in step d) the mixture comprising (i) at least one of an acid and a salt thereof, and ii) a second solvent has a temperature in the range from 17°C to 35°C, further optionally in the range from 20°C to 30°C, still further optionally in the range from 23°C to 27°C, still further optionally in the range from 24-26°C, especially about 25°C.

10. Process for the preparation of polymorph form X of agomelatine according to any of the preceding claims, wherein the providing of agomelatine of formula (A) is or comprises preparing agomelatine, said preparing agomelatine of formula (A) comprising:
providing compound of formula (I) 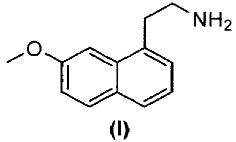
optionally reacting compound of formula (I) with halogenic acid to prepare a halogenic acid salt of compound of formula (I), and
reacting the compound of formula (I) with acetanhydride to obtain agomelatine of formula (A), or
reacting the optionally prepared halogenic acid salt of compound of formula (I) with acetanhydride to obtain agomelatine of formula (A).

11. Process for the preparation of polymorph form X of agomelatine according to claim 10, wherein the providing of compound of formula (I) comprises:
providing a compound of formula (II) 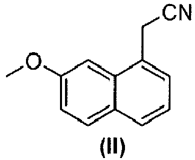 and
reacting the compound of formula (II) with hydrogen to obtain the compound of formula (I).

12. Process for the preparation of polymorph form X of agomelatine according to claim 11, wherein the providing of compound of formula (II) comprises:
providing one or more of: compound of formula (III) and compound of formula (V), 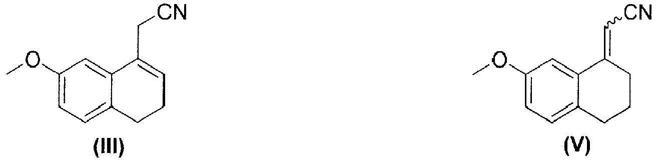
subjecting the one or more of compound of formula (III) and compound of formula (V) to an aromatization reaction to obtain a compound of formula (II).

13. Process for the preparation of polymorph form X of agomelatine according to claim 12, wherein the providing of one or more of: compound of formula (III) and compound of formula (V) comprises:
providing a compound of formula (IV) 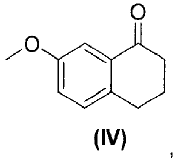
reacting compound of formula (IV) with dialkyl cyanomethylphosphonate, preferably diethyl cyanomethylphosphonate, to obtain one or more of compound of formula (III) and compound of formula (V).

14. Polymorph form X of agomelatine of formula (A) **characterised by** Δ H (J/g) value for form II of below 60, preferably below 30, more preferably below 20, even more preferably below 10, as determined by DSC method at heating rate 1°C/min.

15. Polymorph form X of agomelatine of formula (A) obtainable by a process according to any one of claims 1 to 13.
